# FASCICULE DE BREVET EUROPEEN

(11) **EP 3 768 244 B1**
(45) Date de publication et mention de la délivrance du brevet: **11.05.2022**
(21) Numéro de dépôt: 19717528.4
(22) Date de dépôt: 14.03.2019
(51) Int. Cl.: A61K 9/14, A61K 9/16, A61K 38/54, A61P 1/18

(54) **FORMULATION DE LIBÉRATION INTESTINALE D'ENZYME DIGESTIVE, PROCÉDÉ DE PRÉPARATION ET PRÉPARATION GALÉNIQUE**
FORMULIERUNG EINES VERDAUUNGSENZYMS ZUR FREISETZUNG IM DARM, VERFAHREN ZUR HERSTELLUNG UND GALENISCHE FORM
INTESTINAL-RELEASE FORMULATION OF A DIGESTIVE ENZYME, METHOD OF PRODUCTION AND GALENIC PREPARATION

(30) Priorité: 23.03.2018 FR 1870330
(43) Date de publication de la demande: 27.01.2021
(73) Titulaire: Eneapharm, 31670 Labège (FR)
(72) Inventeur: IOUALALEN, Karim, 31650 SAINT ORENS DE GAMEVILLE (FR); RAYNAL, Rose-anne, 31650 SAINT ORENS DE GAMEVILLE (FR)
(74) Mandataire: BARRE LAFORGUE
(86) Numéro de dépôt international: PCT/FR2019/050557
(87) Numéro de publication internationale: WO 2019/180351

(56) Documents cités:
- EP-A1- 0 436 110
- WO-A1-2014/166994
- FR-A1- 2 913 884
- US-A1- 2011 064 799
- US-A1- 2012 213 857
- US-A1- 2017 112 762
- SONALI RAOSAHEB NAIKWADE ET AL: "Preparation and In Vivo Efficacy Study of Pancreatin Microparticles as an Enzyme Replacement Therapy for Pancreatitis", DRUG DEVELOPMENT AND INDUSTRIAL PHARMACY, vol. 35, no. 4, 1 avril 2009 (2009-04-01), pages 417-432, XP055561443, US ISSN: 0363-9045, DOI: 10.1080/03639040802422104

## Description

L'invention concerne une formulation de libération intestinale d'au moins une enzyme digestive. L'invention vise une telle formulation, dite formulation gastroprotectrice, de protection d'au moins une enzyme digestive au cours de son transit dans l'estomac et de restauration in vivo d'une fonction digestive intestinale.

On connait de FR2913884 un système galénique hydrophobe non ionisable. Le document US 2017/112762 A1 décrit des systèmes galéniques hydrophobes sous formes particules lipidiques solides comprenant entre autre des enzymes et o-enzymes, de taille comprise entre 0,5 et 1500 micromètres et de préférence entre 100 et 400 microns pour administration par voie orale.

La présente invention vise une telle formulation gastroprotectrice, sous forme de particules lipidiques solides, strictement hydrophobes, non hygroscopiques, non injectables, ne contenant pas d'eau, ne contenant pas de tensioactif, ne contenant pas d'agent émulsionnant, ne contenant pas de trace de solvant, ne contenant pas de trace de polymère hydrophile rhéofluidifiant, et contenant au moins une enzyme permettant d'établir au niveau intestinal une activité enzymatique pancréatique exocrine en compensation d'une activité pancréatique exocrine déficiente. Une telle formulation et les formes galéniques destinées à une administration orale d'une telle formulation sont destinées au traitement de l'insuffisance pancréatique exocrine.

Le traitement de l'insuffisance pancréatique exocrine (IPE) repose sur l'administration par voie orale d'enzymes pancréatiques (PERT), ou pancréatine, permettant de pallier l'absence de production d'enzymes digestives exocrine par le pancréas.

La substance médicamenteuse pancrélipase est principalement une combinaison de trois classes d'enzymes: la lipase, la protéase et l'amylase, ainsi que leurs divers co-facteurs et coenzymes. Ces enzymes sont produites naturellement dans le pancréas et sont nécessaires à la digestion des graisses, des protéines et des glucides. La pancrélipase médicamenteuse est typiquement préparée à partir de glandes pancréatiques porcines. D'autres sources de pancrélipase peuvent être utilisées, par exemple celles décrites dans US 6 051 220, US 2004/0057944 et WO2006/044529. Les enzymes catalysent l'hydrolyse des graisses en glycérol et en acides gras, l'amidon en dextrine et en sucres, et les protéines en acides aminés et substances dérivées. Les enzymes pancréatiques natives sont extrêmement sensibles à l'acidité, de sorte que l'apport par voie orale d'enzymes pancréatiques de compensation des enzymes pancréatiques exocrines pose problème. De telles enzymes apportées par voie orale sont dégradées lors de leur passage dans l'estomac. Les lipases sont les plus sensibles à l'inactivation par l'acidité. Pour permettre l'administration par voie orale d'enzymes digestives susceptibles d'atteindre le tractus intestinal, l'industrie a développé différents procédés de formulation, par enrobage polymérique comme décrit dans US8221747 et US8246950.

Les techniques d'enrobage, ou « *polymeric coating* » consistent à élaborer une couche externe de composés isolants, de polymères ou de mélanges, autour du principe actif pour l'isoler du milieu externe comme décrits dans les brevets WO00/30617 et WO02/092106. De nombreux composés polymériques, naturels ou de synthèse ont été utilisés pour la constitution de cette couche externe. On distingue principalement les dérivés de cellulose comme l'hydroxypropylméthylcellulose (HPMC), l'éthylcellulose, les CMC, l'HPMC phtalate, les mélanges de ces produits mais aussi les polymères d'acrylates et leurs dérivés. Cette technique a donné des résultats intéressants pour la préparation de formes à libération retardée et pour la gastroprotection. Une telle formulation dans l'eau reste instable dans le temps, ce qui la rend incompatible avec la préparation de formes aqueuses comme les sirops et les suspensions.

Le document WO 2014/166994 A1 décrit une composition galénique de taille micro/ nanométrique comprenant une structure lipidique et une enzyme (entre autre la pancréatine) et son utilisation pour la libération contrôlée du principe actif par voie orale. Le document US 2012/213857 A1 décrit une pastille sphérique à base exclusivement de pancréatine et de traces d'eau. Le document US 2011/064799 A1 décrit une composition galénique avec un enrobage entérique enzymatique comprenant une enzyme digestive. Le document EP 0 436 110 A1 décrit des petites particules sphériques à base de pancréatine et leur procédé de préparation.

Cependant ces formulations utilisées pour les pancrélipases présentent un certain nombre d'inconvénients comme indiqué dans le document *«* World J. Gastroenterol. 2014 September 7; 20(33): 11467-1148 ». La réponse thérapeutique des produits utilisant ces formulations est faible. La taille moyenne des particules est supérieure à 1,5 mm, induisant une ségrégation avec les aliments dans l'estomac et provoquant un mélange insuffisant dans le tractus intestinal. Enfin la libération des enzymes au niveau intestinal est insuffisante et trop lente.

Il existe donc un besoin pour une formulation gastrorésistante à libération rapide et complète de pancrélipase et/ou d'enzyme pancréatique, dont la taille particulaire soit inférieure à 1,5 mm et qui soit compatible avec une prise facilitée par le patient, en particulier par le patient pédiatrique, pour assurer une meilleure observance du traitement.

L'invention vise à pallier ces inconvénients.

L'invention vise aussi à proposer une nouvelle formulation d'administration d'enzymes digestives permettant :
- une protection des enzymes digestives vis-à-vis des conditions rencontrées dans l'estomac (gastroprotection),
- un masquage du goût, permettant une préparation de formes extemporanées aqueuses, à goût masqué,
- une libération rapide en milieu intestinal,
- un masquage des effets d'irritabilité mucosale.

L'invention concerne une formulation -notamment une formulation de libération intestinale- d'au moins une enzyme digestive, la formulation comprenant des particules lipidiques solides,
- les particules lipidiques solides étant :
- de taille comprise entre 50 µm et 1200 µm (micromètres),
- strictement hydrophobes,
- exemptes d'eau, de solvant organique, de composé tensioactif et de polymère -en particulier de polymère de surface-,
et comprenant :
- au moins une enzyme digestive -notamment au moins une enzyme digestive choisie dans le groupe formé d'une pancrélipase, des enzymes pancréatiques et de leurs analogues-, et
- une matrice cireuse strictement hydrophobe, non hygroscopique,
dans lesquelles, ladite au moins une enzyme digestive ;
- présente une répartition homogène dans chaque particule lipidique solide de la formulation,
- est répartie sans gradient de distribution vers l'intérieur de la matrice cireuse, et
- représente entre 0.1% et 90% de la masse de la formulation,
la formulation présentant un point de fusion compris entre 20°C et 65°C.

L'invention est définie par les revendications.

De façon totalement inattendue, les inventeurs ont découvert que des particules lipidiques solides strictement hydrophobe peuvent être utilisées de façon stable, pour la réalisation d'une formulation à libération rapide d'enzyme(s) digestives(s) pancréatique(s) ou d'analogue(s) d'enzyme(s) pancréatique(s), gastrorésistante -c'est-à-dire permettant une protection des enzymes digestives, et sans que la structure particulaire lipidique solide ne soit sensiblement modifiée lors de la phase gastrique.

La formulation selon l'invention est caractérisée en ce qu'elle a la capacité de protéger, durant la phase gastrique, des enzymes digestives délivrées par voie orale tout en permettant une libération plus complète et rapide dans le tractus digestif intestinal. Malgré la présence de lipase, le système reste stable et fonctionnel tout en masquant le goût et l'odeur.

L'invention concerne une formulation sous forme de particules lipidiques solides, strictement hydrophobes, contenant une ou des enzymes permettant de rétablir au niveau intestinal la fonction enzymatique pancréatique exocrine.

La formulation sous forme de particules lipidiques solides selon l'invention est caractérisée en ce que :
- elle comprend une matrice cireuse strictement hydrophobe et non hygroscopique,
- elle est strictement hydrophobe et gastroprotégée,
- elle est dépourvue de toute trace de solvant aqueux ou organique,
- elle est dépourvue de composés tensioactifs ou amphiphiles ou détergents,
- elle est dépourvue de polymère(s) -en particulier de polymère(s) surfacique(s)-et de résidu(s) de tel(s) agent(s) polymérique(s). Les particules lipidiques solides sont élaborées sans avoir recours à l'utilisation de tels polymères,
- elle est caractérisée par une répartition homogène de ladite au moins une enzyme digestive au sein de la matrice cireuse sans gradient radial,
- est biocompatible et dispersible en milieu intestinal,
- est non administrable par voie parentérale,
- contient ladite au moins une enzyme digestive -notamment une pancrélipase ou une ou une pluralité d'enzymes pancréatiques ou des extraits pancréatiques, ou des enzymes analogues seules ou en mélanges- et éventuellement des cofacteurs et coenzymes et,
- elle permet outre la gastroprotection, d'améliorer la stabilité de ladite au moins une enzyme digestive, d'accélérer la libération intestinale en assurant un masquage du goût et de l'odeur.

Avantageusement, la formulation est constituée uniquement de particules lipidiques solides, les particules lipidiques solides étant formées uniquement d'au moins une enzyme digestive et d'une matrice cireuse strictement hydrophobe, non hygroscopique.

Avantageusement, les particules lipidiques solides présentent une taille comprise entre 150 µm et 800 µm, notamment comprise entre 250 µm et 550 µm. Elles sont strictement hydrophobes, contenant une ou des enzymes permettant de rétablir au niveau intestinal la fonction digestive.

Avantageusement, la formulation présente un point de fusion compris entre 20°C et 65°C, notamment un point de fusion compris entre 20°C et 55°C, de préférence compris entre 30°C et 50°C, encore plus préférentiellement compris entre 32°C et 48°C.

Avantageusement, la matrice cireuse présente un point de fusion compris entre 20°C et 65°C, notamment un point de fusion compris entre 20°C et 55°C, de préférence compris entre 30°C et 50°C, encore plus préférentiellement compris entre 32°C et 48°C.

Par la suite dans le texte, les expressions, « particule lipidique » et « granule lipidique », s'entendront comme ayant la même signification.

On choisit une matrice cireuse présentant une composition appropriée, compatible en termes de toxicité, de biocompatibilité, de non immunogénicité et de biodégradabilité avec une absorption par voie orale. Les composants de la matrice cireuse seront choisis parmi les composants déjà utilisés pour l'administration par voie orale, tels que ceux définis dans la liste GRAS éditée par la « Food and Drug Administration », et de telle sorte que les particules lipidiques solides formées présentent des propriétés d'incorporation, de masquage de goût, de stabilisation et de libération de ladite au moins une enzyme digestive.

Au moins une enzyme digestive est la pancrélipase également appelée pancréatine. Dans tout le texte, les termes « pancrélipase » ou « pancréatine » désignent un mélange de plusieurs types d'enzymes, les enzymes amylase, lipase et protéase. Une pancrélipase ou pancréatine peut être obtenue par extraction à partir du pancréas. Elle peut être produite artificiellement, ou obtenue à partir de sources autres que le pancréas, par exemple à partir de microbes, de plantes ou d'autres tissus animaux. Lorsqu'elle provient d'un extrait pancréatique, la pancrélipase est associée à des cofacteurs. La pancrélipase est disponible dans le commerce, par exemple chez *Nordmark Arzneimittel* ou *Scientific Protein Laboratories.*

Avantageusement et selon l'invention, ladite au moins une enzyme digestive formulée est stabilisée.

Dans un mode de réalisation d'une formulation selon la présente invention, ladite au moins une enzyme digestive comprend une lipase. Le terme « lipase » désigne une enzyme qui catalyse l'hydrolyse des lipides en glycérol et en acides gras simples.

Des exemples de lipases convenables pour la mise en oeuvre de la présente invention comprennent une lipase animale par exemple une lipase d'origine porcine ou bovine, une lipase bactérienne -par exemple une lipase de *Pseudomonas* (« *Pseudomonas* lipase »), une lipase fongique, une lipase végétale, une lipase recombinante, une lipase chimiquement modifiée, ou un mélange de celles-ci.

Dans un mode de réalisation d'une formulation selon la présente invention, au moins une enzyme digestive formulée comprend une amylase. Le terme « amylase » désigne des enzymes glycosides hydrolases qui décomposent l'amidon, par exemple les α-amylases, les β-amylases, les γ-amylases, les α-glucosidases acides, les amylases salivaires.

Les amylases utilisables dans une formulation selon la présente invention sont choisies dans le groupe formé des amylases animales, des amylases bactériennes, des amylases fongiques, par exemple, une amylase *d'Aspergillus,* une amylase *d'Aspergillus oryzae,* des amylases végétales, des amylases chimiquement modifiées, ou des mélanges de celles-ci.

Dans un mode de réalisation d'une formulation selon la présente invention, au moins une enzyme digestive est une protéase. Le terme « protéase » désigne les enzymes par exemple des protéinases, des peptidases ou des enzymes protéolytiques, qui rompent les liaisons peptidiques entre les acides aminés des protéines. Les protéases sont généralement identifiées par leur type catalytique, par exemple les peptidases d'acide aspartique, les cystéine(thiol)-peptidases, les sérine-peptidases, les thréonine-peptidases, les protéases alcalines ou semi-alcalines, les protéases neutres et les nouvelles peptidases.

À titre d'exemples de protéases utilisables dans une formulation selon la présente invention, on peut citer les sérine-protéases, les thréonine-protéases, les cystéine-protéases, les protéases d'acide aspartique, et ce sans limitation de type de protéases. Une formulation selon la présente invention peut comprendre au moins une protéase animale, au moins une protéase bactérienne, au moins une protéase fongique, au moins une protéase végétale, au moins une protéase recombinante, au moins une protéase chimiquement modifiée, seules ou leurs mélanges.

Dans la description on désigne comme enzymes recombinantes, les enzymes produites par recombinaison d'ADN par une cellule hôte appropriée, choisie parmi l'une quelconque des cellules hôtes de bactéries, de levures, de champignons, de plantes, animales, ou des enzymes recombinantes comprenant une séquence d'acides aminés homologue ou substantiellement identique à une séquence naturelle d'enzymes ou comprenant un acide nucléique homologue ou sensiblement identique à un acide nucléique codant pour l'enzyme naturelle.

Les formulations selon la présente invention peuvent comprendre une ou plusieurs lipase(s), une ou plusieurs amylase(s), une ou plusieurs protéase(s), des mélanges comprenant un ou deux ou chacun des différents types d'enzymes.

Dans un mode de réalisation, ladite au moins une enzyme digestive est un extrait pancréatique porcin ou bovin comprenant diverses lipases par exemple, une lipase, une colipase, une phospholipase A2, une cholestérol estérase, des protéases, par exemple une trypsine, une chymotrypsine, une carboxypeptidase A et/ou B, une élastase, une kininogénase, un inhibiteur de la trypsine, des amylases, et éventuellement des nucléases -ribonucléase, désoxyribonucléase-. Dans un mode de réalisation, ladite au moins une enzyme digestive est un mélange sensiblement similaire au fluide pancréatique humain. Dans encore un autre mode de réalisation, au moins une enzyme digestive est la pancrélipase USP.

Les activités lipasiques dans les compositions et les formes de la présente invention peuvent être d'environ 100 à 100 000 UI, et dans une mise en oeuvre particulière de 500 à 25000 UI par dose unitaire administrable.

Les activités d'amylase dans les compositions selon l'invention sont comprises entre 100 et 320000 UI par unité thérapeutique ou dose unitaire, et préférentiellement entre 1500 et 75 000 UI.

Les activités de protéase dans les compositions ou les formes de la présente invention peuvent être comprises entre 100 et 180 000 UI, et préférentiellement entre 1000 et 80000 UI par dose unitaire.

Dans un mode de réalisation, l'activité de la lipase est comprise entre environ 400 et 500 UI, l'activité de l'amylase est comprise entre environ 1750 et 2750 UI et l'activité de la protéase est comprise entre environ 1200 et 2000 UI.

Dans un mode de réalisation, l'activité de la lipase est comprise entre environ 4000 et 5000 UI, l'activité de l'amylase est comprise entre environ 17500 et 27500 UI et l'activité de la protéase est comprise entre environ 12000 et 20000 UI.

Dans un autre mode de réalisation, l'activité de la lipase est comprise entre environ 10 000 et 12500 UI, l'activité de l'amylase est comprise entre environ 22 000 et 90 000 UI et l'activité de la protéase est comprise entre environ 17 000 et 75 000 UI.

Dans encore un autre mode de réalisation, l'activité lipase est comprise entre environ 12 500 et 15 500 UI, l'activité de l'amylase est comprise entre environ 25000 et 90000 UI et l'activité de la protéase est comprise entre environ 26000 et 100000 UI par dose unitaire.

Dans encore un autre mode de réalisation, l'activité lipase est comprise entre environ 17000 et 25000 UI, l'activité de l'amylase est comprise entre environ 40000 et 185000 UI et l'activité de la protéase est comprise entre environ 30000 et 125000 UI par dose unitaire.

Ainsi, l'invention a pour objet une formulation d'enzymes digestives dans une matrice cireuse sous forme de particules lipidiques solides, permettant de rétablir au niveau intestinal la fonction enzymatique pancréatique exocrine.

La matrice cireuse des particules lipidiques solide d'une formulation selon l'invention, est constituée d'un composé hydrophobe ou d'un mélange de composés hydrophobes insoluble(s) dans l'eau, solide(s) à température ambiante et totalement dépourvu(s) de composés tensioactifs, de résidus de solvant et d'eau. Ainsi toute réaction d'hydrolyse ou d'oxydation est évitée. À titre d'exemple, on peut citer les cires hydrophobes ou les mélanges de ces cires hydrophobes, les cires végétales, les cires animales, les cires synthétiques et/ou minérales. La matrice cireuse peut comprendre au moins une huile et au moins un composé hydrophobe permettant d'ajuster le point de fusion, la dureté, les propriétés physicochimiques et les propriétés biologiques comme la biodégradabilité. Les particules peuvent contenir en outre des additifs, des actifs solubles ou insolubles comme des particules minérales. Selon l'invention, on utilise à titre de matrice cireuse des mélanges dont le point de fusion est compris entre 20°C et 65°C, préférentiellement entre 32°C et 48°C.

On peut utiliser des triglycérides d'acides gras de C8 à C30, des triglycérides fractionnés, des triglycérides modifiés, des triglycérides synthétiques, des mélanges de triglycérides, des triglycérides à chaîne moyenne et à longue chaîne et des triglycérides structurés. Il est aussi possible d'utiliser d'autres cires comme des alcools gras de haut poids moléculaire, des acides gras à chaine hydrocarbonée préférentiellement linéaire et saturée, des acides gras à nombre pair d'atomes de carbone de C12 à C30, des esters d'acides et d'alcools de haut poids moléculaire, notamment des esters d'acide gras de C8 à C30 et d'alcool de C2 à C32. Dans tous les cas, le mélange obtenu est solide -notamment à température ambiante- et caractérisé par l'absence de composés tensioactifs, par un comportement hydrophobe, une non mouillabilité par l'eau et l'absence d'hygroscopicité. Les acides gras utilisables dans la présente invention sont des acides gras non neutralisés et non ionisés (c'est-à-dire exclusivement sous forme acide carboxylique -COOH).

Avantageusement, la matrice cireuse comprend -notamment est constituée de- au moins un corps gras choisi dans le groupe formé des cires végétales - notamment de la cire de carnauba, de la cire de candelilla, de la cire d'alfa, de la cire d'olivier, de la cire de riz, de la cire de jojoba, de la cire de jojoba hydrogénée et des cires absolues de fleurs-, des cires d'abeilles, des cires d'abeilles modifiées, de la paraffine et des dérivés de paraffine, d'ozokérite, des polyoléfines, des acides gras non neutralisés et non ionisés (c'est-à-dire exclusivement sous forme acide carboxylique - COOH), des esters d'acides gras -notamment choisis dans le groupe formé des esters d'acides gras à chaînes linéaires ayant un nombre d'atomes de carbone compris entre 4 et 30, notamment des esters d'acide laurique, des esters d'acides myristique, des esters d'acide palmitique et des esters d'acide stéarique-, de triglycérides, de dérivés de triglycérides, d'huile de palme, du beurre de cacao.

La matrice cireuse comprend -notamment est constituée exclusivement de- au moins une cire choisie dans le groupe formé des triglycérides et des dérivés suivants sans que la liste soit limitative: tri-behenate, Glyceryl tributyrate, Glyceryl tricaproate, Glyceryl tricaprylate, Glyceryl tricaprate, Glyceryl triundecanoate, Glyceryl trilaurate, Glyceryl trimyristate, Glyceryl tripalmitate, Glyceryl tristearate, Glyceryl trimyristoleate, Glyceryl tripalmitoleate, Glyceryl trioleate, Glyceryl trilinoleate, Glyceryl trilinolenate, Glyceryl tricaprylate/caprate, Glyceryl tricaprylate/caprate/laurate, Glyceryl tricaprylate/caprate/linoleate, Glyceryl tricaprylate/caprate/stearate, Glyceryl tricaprylate/laurate/stearate, Glyceryl 1,2-caprylate-3-linoleate, Glyceryl 1,2-caprate-3-stearate, Glyceryl 1,2-laurate-3-myristate, Glyceryl 1,2-myristate-3-laurate, Glyceryl 1,3-palmitate-2-butyrate, Glyceryl 1,3-stearate-2-caprate, Glyceryl 1,2-linoleate-3-caprylate. On peut aussi citer l'huile de palme, la cire de Carnauba, la cire de *Candelilla,* la cire *d'Alfa,* le beurre de cacao, l'ozokérite, les cires végétales comme la cire d'olivier, la cire de riz, la cire de jojoba hydrogénée ou les cires absolues de fleurs. Les cires d'abeilles et cires d'abeilles modifiées peuvent aussi être utilisées comme les cires d'origine naturelle. Les acides gras pouvant être utilisés selon l'invention sont sous forme acide (c'est-à-dire sous forme acide carboxylique -COOH) tels que par exemple l'acide myristique, l'acide laurique, l'acide palmitique ou encore l'acide stéarique. Leurs sels formant des savons, ne peuvent en aucun cas être utilisés car ils favoriseraient la mouillabilité de la matrice cireuse.

Pour améliorer la solubilité ou la dispersion de ladite au moins une enzyme digestive dans la matrice, il est parfois nécessaire d'ajouter une huile. Elle permet de pouvoir ajuster le point de fusion. On peut citer aussi les triglycérides huileux comme les huiles végétales, certaines huiles de poisson, huiles végétales hydrogénées, les huiles végétales partiellement hydrogénées.

Les composés commerciaux pharmaceutiques désignés comme "Fat hard" constitués essentiellement de triglycérides peuvent aussi être utilisés. Des traces résiduelles de mono- et diglycérides pouvant être présentes dans ces derniers, il convient alors de vérifier que le comportement strictement hydrophobe et non mouillable est bien respecté pour pouvoir utiliser ces produits comme matière première de la matrice. Tel est le cas des produits dénommés suppocires^{®} AM, CM, DM, et D.

Outre les cires mentionnées ci-dessus, la matrice cireuse de particules lipidiques solides selon l'invention peut contenir une huile ou un mélange, par exemple une huile de silicone hydrophobe, du squalène, l'un de ses dérivés et ses esters.

D'autres composés huileux comme l'alcool oléique, l'huile de tournesol, l'huile de palme, l'huile d'olive, les acides gras non neutralisés et non ionisés (c'est-à-dire sous forme acide carboxylique -COOH) et alcools gras peuvent être utilisés, mais le mélange obtenu doit être caractérisé par un comportement hydrophobe, une absence de miscibilité avec l'eau. L'homme de l'art sait que pour les composants de la matrice cireuse, la température de fusion par chauffage de la matrice cireuse ne doit pas dépasser la température de dégradation des différents composés.

D'autres composés peuvent être ajoutés à la matrice cireuse comme les paraffines. On peut citer les agents de charges, comme le talc, le kaolin ou le mica, les colorants, les agents permettant d'ajuster l'aspect, la couleur, la densité et la dureté de la matrice cireuse. La matrice cireuse contient une enzyme digestive ou un mélange d'enzymes digestives pouvant être sous une forme dispersée ou sous une forme solubilisée dans la matrice cireuse ou présenter les deux formes.

Selon un mode de réalisation particulier de l'invention, la matrice cireuse peut être formée d'un mélange de cires.

Avantageusement et selon l'invention, la formulation contient des acides gras (non neutralisés et non ionisés, c'est-à-dire sous forme acide carboxylique -COOH) ou des esters d'acides gras en proportion massique comprise entre 0,5% et 75% de la formulation, préférentiellement comprise entre 1% et 30% de la formulation. Dans tout le texte, l'expression « acide(s) gras » signifie un(des) acide(s) gras non neutralisé(s) et non ionisé(s) (c'est-à-dire sous forme acide carboxylique -COOH).

Avantageusement et selon l'invention, chaque particule lipidique solide comprend une seule enzyme digestive.

Avantageusement et selon l'invention, la formulation est formée d'un mélange de particules lipidiques solides, chaque particule lipidique solide comprenant une seule enzyme digestive, le mélange de particules lipidiques solides formant un mélange d'enzymes digestives.

L'invention concerne aussi une préparation galénique comprenant une formulation selon l'invention.

Avantageusement, la préparation galénique comprend une formulation selon l'invention et au moins un composé additionnel choisi dans le groupe formé des agents de lubrification -notamment du talc- des agents d'homogénéisation -notamment de la silice-, des liants pharmaceutiquement acceptables, des stabilisants, des délitants, des colorants, des conservateurs, des édulcorants, des épaississants -notamment au moins un dérivé de cellulose-.

Avantageusement, ledit composé additionnel est en proportion massique comprise entre environ 0,1% et 99% dans la composition galénique, préférentiellement comprise entre 3% et 32%.

Avantageusement, la préparation galénique se présente sous une forme choisie dans le groupe formé d'une poudre -notamment d'une poudre permettant de reconstituer une dispersion par addition d'eau, ladite poudre étant contenue dans un contenant unitaire ou multiple se présentant sous la forme de sachet, ou de flacon en verre ou en polymère ou métallique-, d'un comprimé, d'une tablette -notamment d'une tablette obtenue par lyophilisation-, d'une gélule, d'une capsule -notamment d'un comprimé, d'une tablette, d'une gélule ou d'une capsule conditionnée sous blister ou en sac de conditionnement.

On désigne par « mélange contenant le composé actif » ou « matrice contenant l'actif » ou « produit contenant l'actif », le résultat du mélange des constituants de la matrice cireuse, d'excipients hydrophobes et de ladite au moins une enzyme digestive après fusion.

L'invention concerne aussi un procédé de préparation d'une formulation selon l'invention.

Dans une mise en oeuvre préférentielle, la formulation est préparée selon un procédé comprenant les étapes suivantes :
- a/ fusion de la matrice cireuse sous agitation, puis diminution de la température de la matrice cireuse à une température supérieure d'au moins 3°C à la température de fusion de la matrice cireuse,
- b/ addition et dispersion de ladite au moins une enzyme digestive dans la matrice cireuse fondue,
- c/ solidification du mélange formé par refroidissement à une température inférieure d'au moins 15°C à la température de de fusion de la matrice cireuse,
- e/ broyage mécanique à une température inférieure d'au moins 10°C, et préférentiellement 20°C, au point de fusion du mélange solidifié (de la matrice cireuse contenant ladite au moins une enzyme digestive), ce par quoi on forme la formulation de particules lipidiques solides sous forme de poudre.

Avantageusement, le procédé selon l'invention comprend une étape d/ de prébroyage du mélange refroidi formé à l'issue de l'étape c/.

Dans un procédé selon l'invention, lors de l'étape a/, on réalise une préparation de la matrice hydrophobe par fusion des excipients puis agitation et diminution de la température à au moins 3°C, préférentiellement 5°C, au-dessus de la température de fusion du mélange final.

Lors de l'étape b/, on réalise une addition et une dispersion de l'enzyme digestive dans la matrice fondue.

Lors de l'étape c/, on réalise une récupération et une solidification de la matrice par refroidissement à au moins 15°C en dessous du point de fusion du mélange obtenu.

Lors de l'étape d/, on réalise un prébroyage.

Lors de l'étape e/, on réalise un broyage à une température d'au moins 10°C et préférentiellement 20°C sous le point de fusion de la matrice.

Lors de l'étape f/, on réalise une récupération de la poudre de pancrélipase broyée.

Dans une étape b/ d'un procédé selon l'invention, ladite au moins une enzyme digestive est dispersée à titre de principe actif dans une cire ou un mélange de cires et d'excipients strictement hydrophobes appelés matrice cireuse hydrophobe préalablement fondue. Ladite au moins une enzyme digestive est protégée de tout contact avec l'oxygène et l'eau et plus généralement de tout stress chimique externe. On réalise l'addition de ladite au moins une enzyme digestive dans la matrice cireuse à une température de la matrice cireuse supérieure -notamment de au minimum 3°C, de préférence 5°C- au point de fusion de la matrice cireuse, mais toujours en dessous de la température de dégradation ou de désactivation de ladite au moins une enzyme digestive. La matrice cireuse liquide obtenue, contenant de ladite au moins une enzyme digestive, est alors solidifiée par refroidissement puis prébroyée.

Le mélange solidifié est ensuite broyé afin d'obtenir une poudre de granulométrie contrôlée et prête à l'emploi. Cette poudre de particules lipidiques solides peut être dispersée dans l'eau sans risque pour la(les) enzyme(s) digestive(s) en raison de sa nature strictement hydrophobe.

Ce mode de réalisation de la formulation est donc rapide et ne nécessite pas de modifications chimiques de la(des) enzyme(s) digestive(s) ou de traitement de surface des particules lipidiques solides ou des cristaux de la(des) enzyme(s) digestive(s). Il permet d'incorporer la(les) enzyme(s) digestive(s) à la formulation dès la première phase de mélange des différents constituants de la formulation. Il est peu onéreux et facile à mettre en oeuvre. La mise en oeuvre du procédé de préparation d'une formulation selon la présente invention, ne fait pas intervenir d'addition d'agents émulsionnants ni de produits amphiphiles, et ne nécessite pas non plus de solvants organiques dont l'élimination reste toujours difficile et dont l'emploi est de plus en plus restrictif. Il ne fait pas non plus intervenir d'agent rhéofluidifiant. Il ne fait intervenir aucun contact avec de l'eau ni avec toute composition aqueuse au cours du procédé, en évitant toute solubilisation d'enzyme(s) digestive(s) hydrosolubles dans cette composition aqueuse et toute extraction d'enzyme(s) digestive(s) hydrosolubles. La(les) enzyme(s) digestive(s) est(sont) uniformément répartie(s) dans la matrice cireuse des particules lipidiques solides, même en surface desdites particules, sans gradient -notamment sans gradient radial- de concentration.

Le mélange des différents composants de la matrice cireuse et de ladite au moins une enzyme digestive est réalisé dans un réacteur thermostaté ou un fondoir. On fait fondre en premier le composant de la matrice cireuse présentant le point de fusion le plus élevé, sous agitation mécanique appropriée à la dispersion de tous les composants.

Ce procédé est donc rapide et ne nécessite pas d'étape d'agitation longue et délicate.

Le mélange est alors refroidi immédiatement afin de protéger la(les) enzyme(s) digestive(s) sensible(s) et obtenir une phase solide formée de la matrice cireuse et de la(des) enzyme(s) digestive(s). Selon une mise en œuvre particulière de l'invention, pour les quantités inférieures à 1 kg, le refroidissement de la matrice cireuse fondue contenant la(les) enzyme(s) digestive(s) dispersée(s) peut être réalisé par étalement sur des plateaux à refroidissement de contact. La solidification est obtenue en moins de 120 secondes, puis les plateaux sont placés en chambre froide ce qui permet de découpler les étapes si nécessaire.

Pour des quantités plus importantes le refroidissement peut être réalisé par passage sur un système de refroidissement continu. Le produit fondu est déposé sous forme de gouttelettes, de filaments ou de film sur un convoyeur à bande d'acier inoxydable traversant une chambre de refroidissement et est récupéré sous forme solide en sortie de la chambre de refroidissement. D'autres systèmes comme ceux équipés de cylindre réfrigéré, peuvent être utilisés.

La température de refroidissement de la matrice est contrôlée à au moins 15°C en dessous de la température de fusion du mélange. Selon une forme de réalisation de l'invention, la température de refroidissement est comprise entre -195°C et 30°C et préférentiellement entre -10°C et 5°C.

La matrice cireuse est ensuite refroidie par contact ou convection avant d'être déversée dans le système de prébroyage afin d'obtenir des fragments préférentiellement de taille inférieure à 40 mm, pour pouvoir alimenter correctement le broyeur. À cet effet on peut utiliser des prébroyeurs ou tout autre système approprié. Certains mélanges solidifiés sont fragiles et peuvent être prébroyés et broyés dans le même appareil en une seule étape mais à des vitesses différentes.

Avantageusement et selon l'invention, au moins l'une des étapes c) de solidification, d) de prébroyage et e) de broyage est réalisée par refroidissement avec de la carboglace^{®} ou l'azote liquide.

Dans une dernière étape selon l'invention, la matrice prébroyée est réduite en poudre par broyage. Un grand nombre de broyeurs peuvent être utilisés comme les broyeurs à marteaux, à couteaux, à rotor, à billes ou à jets. Pour faciliter le broyage de la matrice cireuse, une mise en oeuvre consiste à refroidir la matrice pour la durcir et la fragiliser. Cette opération intervient avant ou au cours de l'étape de broyage.

La poudre de particules lipidiques solides comprenant la matrice cireuse obtenue à l'issue du broyage peut être conditionnée directement. Cette poudre selon l'invention présente une granulométrie comprise entre 50 et 1200 microns (µm) et préférentiellement entre 150 et 800 microns (µm). Dans une mise en oeuvre préférentielle la poudre a une granulométrie comprise entre 250 et 550 microns (µm).

La préparation de la formulation selon l'invention ne fait pas intervenir d'étape de dispersion d'une matrice cireuse dans une phase aqueuse liquide ou gélifiée. La formulation selon l'invention et les particules lipidiques solides sont dépourvues d'eau de surface et en profondeur, et de tout résidu de polymère -en particulier de polymère(s) surfacique(s)- hydrosoluble, en particulier rhéofluidifiants, susceptible d'interagir avec ladite au moins un enzyme digestive et d'en affecter la stabilité physicochimique, et de diminuer la durée de conservation et le taux de chargement.

L'invention s'étend aussi à une préparation galénique comprenant une formulation selon l'invention. Avantageusement et selon l'invention, la préparation galénique contient au moins un composé additionnel choisi dans le groupe formé des agents de lubrification des agents d'homogénéisation, des liants pharmaceutiquement acceptables, des stabilisants, des délitants, des colorants, des conservateurs, des édulcorants, des épaississants.

Avantageusement et selon l'invention, ledit composé additionnel est en proportion massique comprise entre environ 0,1 et 99% dans la composition galénique. La capacité de chargement de la matrice cireuse peut s'étendre de 0,1 % à 90 % par rapport au poids. L'homme de l'art sait que lorsqu'on effectue l'incorporation de ces composants dans la matrice cireuse selon l'invention, il convient de choisir une composition cireuse hydrophobe appropriée de telle sorte que le procédé puisse être mis en œuvre.

La préparation galénique peut être présentée sous une forme galénique classique comme une poudre, une gélule, une capsule, un comprimé, un comprimé -notamment un comprimé orodispersible-, une tablette -notamment une tablette issue de lyophilisation-, une suspension aqueuse. Elle peut être présentée sous la forme d'une poudre dispersible conditionnée en sachet ou en flacon permettant d'obtenir une forme liquide.

Avantageusement, la préparation galénique contient au moins un composé additionnel ou additif choisi dans le groupe formé des agents de lubrification -par exemple le talc-, des agents d'amélioration de l'homogénéité -comme la silice-, des liants pharmaceutiquement acceptables, des stabilisants, des délitants, des désintégrants, des colorants, des conservateurs, des édulcorants, des diluants, des épaississants -comme les dérivés de cellulose-.

Des exemples de liants appropriés comprennent les amidons, les sucres comme le lactose, les alcools de sucre, le xylitol, le sorbitol, maltitol, la cellulose, les celluloses modifiées l'hydroxypropylcellulose (HPC), la carboxyméthylcellulose (CMC) sodique, l'acide alginique, le polyvinylpyrrolidone (PVP) et leurs mélanges.

À titre d'exemples de désintégrants appropriés, on peut citer le phosphate de calcium dibasique, l'acide alginique, l'HPC, la CMC, les résines échangeuses d'ions gonflables, les alginates, la formaldéhyde-caséine, la cellulose, la croscarmellose sodique, la crospovidone, la cellulose, le carboxyméthylamidon sodique, le glycolate d'amidon et leurs mélanges. Des exemples de lubrifiants appropriés comprennent le stéarate de calcium, le stéarate de magnésium, le stéarylfumarate de sodium, l'acide stéarique, le stéarate de zinc, le talc, les cires, Sterotex^{®}et leurs mélanges, des exemples d'agents favorisant l'homogénéité des poudres comme le dioxyde de silicium colloïdal, le talc et leurs mélanges.

À titre d'exemple on peut citer comme diluant, la cellulose microcristalline, par exemple le Pharmacel 112 de DFE Pharma, l'amidon, le phosphate de calcium, le lactose comme le Pharmatol, le saccharose, le carbonate de magnésium le mannitol, le sorbitol et leurs combinaisons. Dans un autre mode de réalisation, les compositions ou les formes posologiques orales de la présente invention peuvent comprendre une combinaison de diluants tels que la cellulose microcristalline, l'amidon et le lactose.

La proportion massique de diluant peut être comprise entre environ 0,1% et 99% et préférentiellement entre 3% et 32% de la préparation galénique.

Dans un autre mode de réalisation, les compositions ou formes posologiques orales de la présente invention peuvent comprendre une combinaison de délitants tels que la cellulose microcristalline et le glycolate d'amidon sodique ou la croscarmellose sodique et la crospovidone.

La proportion massique de désintégrant peut être comprise entre environ 0,1% et 25%, préférentiellement entre 0,5% et 6% de la préparation galénique.

La poudre obtenue selon l'invention peut être conditionnée dans des formes unitaires ou multiples par exemple sous forme de sac, de flacon, sachet et de blister.

D'autres buts, caractéristiques et avantages de l'invention apparaîtront à la lecture de la description suivante de certains de ses modes de réalisation possibles et des exemples donnés à titre non limitatif.

Les exemples qui suivent illustrent l'invention. Pour certains des exemples suivants, les tests de masquage de goût et d'odeur ont été réalisés auprès d'un échantillon de 10 individus. Les produits testés ne sont jamais absorbés.

Les résultats sont exprimés selon l'échelle suivante :
- 1 : le goût du principe actif n'est pas détecté ;
- 2 : le goût du principe actif est légèrement perçu ;
- 3 : le goût du principe actif est détecté ;
- 4 : le goût du principe actif est encore acceptable ;
- 5 : le goût du principe actif n'est pas acceptable.

La valeur du test est calculée en réalisant la moyenne des notes obtenues sur un total maximal de 10 (/10).

On détermine l'activité lipasique selon la méthode de la « Fédération Internationale Pharmaceutique / Pharmacopée Européenne » (abrégée ci-après FIP / Ph.Eur.). Dans cette méthode d'analyse standard, l'activité hydrolytique de la lipase dans l'échantillon à étudier est déterminée en utilisant l'huile d'olive (USP) à titre de substrat. Les acides gras libres libérés à partir des triglycérides de l'huile d'olive sont titrés avec une solution d'hydroxyde de sodium à pH constant de 9,0. L'activité lipasique de l'échantillon est déterminée en comparant la vitesse à laquelle l'échantillon hydrolyse une émulsion d'huile d'olive avec la vitesse à laquelle une suspension d'une poudre de référence de pancréas standard hydrolyse le même substrat dans les mêmes conditions.

Exemple 1 : Préparation d'une formulation de pancréatine stabilisée à goût masqué, gastroprotégée à libération rapide.

Composition :

| | |
|---|---|
| - Triglycérides PPM1 (Stéarinerie Dubois) | 0.94 kg |
| -cire | 0.1 kg |
| - pancréatine | 1,625 kg |
| - Talc | 0.020 kg |
| - Silice | 0.005 kg |

Dans un réacteur thermostaté de 5 litres on porte le composé de plus haut point de fusion, 5°C au dessus de sa température de fusion, puis on ajoute progressivement les différents composés du point de fusion le plus élevé au moins élevé. La température du mélange est progressivement abaissée pour être maintenue à 3°C au dessus de la température de fusion du nouveau mélange obtenu, ici 48°C. On ajoute en dernier la pancréatine. La dispersion de la pancréatine dans la matrice cireuse fondue, est réalisée à l'aide d'un système d'agitation équipé d'un mobile en forme d'ancre à une vitesse de 200 tours/min. Puis on applique une agitation par turbine turrax T25 à 4500 tours/min pendant 6 min pour obtenir une dispersion complète.

La matrice cireuse contenant la pancréatine est solidifiée par écoulement sur cylindre rotatif équipé d'un racloir de décrochage, dont la température est de 0°C.

Les fragments récupérés d'une taille moyenne de 4 millimètres sont alors refroidis par carboglace^{®}. Le mélange est prébroyé puis broyé à l'aide d'un broyeur à couteaux de type Retsch GM Inox.
Vitesse : 3000 (tours/min)
Durée : 90 secondes
Granulométrie : diamètre moyen de 565 microns (µm) ;

Cette poudre comprenant de la pancréatine est ensuite évaluée par test de goût. Le résultat est une valeur moyenne de 1.20 au test de goût.

La valeur moyenne étant inférieure à 2 ; la saveur et l'odeur de pancréatine ne sont pas détectables et aucune sensation d'irritation mucosale n'est constatée.

Exemple 2 : Préparation de poudre contenant de la pancréatine gastroprotégée, stabilisée à goût masqué et libération intestinale accélérée. Cette formulation est destinée à l'élaboration d'un produit dédié au traitement de l'insuffisance pancréatique exocrine.

Composition :

| | |
|---|---|
| - Triglycérides PPM1 (Stéarinerie Dubois) | 0,69 kg |
| - Acide stéarique | 0,058 kg |
| - Pancréatine | 1,325 kg |
| - Talc | 0.020 kg |
| - Silice | 0.005 kg |
| - Arôme de menthe | 0.009 kg |

La poudre est préparée selon le protocole décrit à l'exemple 1. La poudre obtenue est caractérisée par l'absence de détection de la saveur de pancréatine avec un score de 1.2 au test de goût.

La poudre obtenue est caractérisée par une saveur marquée de menthe avec un score de 5 au test de goût.

Exemple 3 : Préparation d'une poudre hydrodispersible, contenant des particules chargées en enzymes digestives pour la voie orale.

Composition :

| | |
|---|---|
| - Particules selon l'exemple 1 | 100 g |
| - Arôme fruit rouge | 8 g |
| - Aspartame | 4 g |
| - Gomme de xanthane (Xanthural 180) | 1 g |
| - Talc | 0.5 g |
| - Silice colloidale | 0.1 g |

Dans un mélangeur à poudre de type Turbula (WAB France), on place les constituants de la composition. Après mélange, la poudre est répartie en sachet unitaire de 0.25 g. La reprise par 50 ml d'eau permet de reconstituer une dispersion aqueuse. Le test de goût réalisé sur la dispersion ne donne aucune détection du principe actif.

Exemple 4 : Préparation de particules contenant de la pancrélipase

Composition :

| | |
|---|---|
| - Mélange de Triglycérides (MP 45°C) | 700 g |
| - Triglycérides DUB PP(Stéarinerie Dubois) | 100 g |
| - Pancréatine | 1600 g |
| - Talc | 10 g |
| - Hydrogénocarbonate de sodium | 5g |
| - Silice (Aérosil) | 2g |

Dans un réacteur thermostaté de 500 ml, on porte le composé de plus haut point de fusion les triglycérides DUB PP à 50°C, puis on ajoute progressivement les différents composés du point de fusion le plus élevé au moins élevé. La température du mélange est progressivement abaissée pour être maintenue à 45 °C. Pendant l'addition de la composition, la vitesse d'agitation de l'hélice tripale est de 100 tours/min.

On ajoute en dernier la pancréatine. La dispersion de la pancréatine dans la phase lipidique, est réalisée à l'aide d'un système d'agitation de type turbine de marque Turrax T25 à une vitesse de 6000 tours/min. La matrice est solidifiée par écoulement sur tambour rotatif inox de diamètre 55 cm dont la température est portée à -10°C, tournant à 5 tours/min et équipé d'une lame tangentielle destinée à décrocher la matrice solidifiée en surface. Les fragments récupérés d'une taille moyenne de 25 millimètres sont alors prébroyés puis broyés à l'aide d'un broyeur à couteaux type Retsch GM200 cochargé en glace carbonique selon les conditions :
- Prébroyage : 20 secondes à 1500 tours/min
- Broyage : 70 secondes à 3000 tours/min

Les particules ainsi obtenues ont une taille moyenne de 482 microns (µm).

Essai de stabilité en solution d'acide chlorhydrique 0,1N : La stabilité de la formulation est déterminée en suivant l'évolution de l'activité de la lipase en fonction du temps. Les résultats sont exprimés en pourcentage d'activité initiale en fonction du temps et sont rassemblés dans le tableau 1 ci-dessous. L'activité lipasique est déterminée après neutralisation à pH 7 par addition de solution NaOH 0,02N.

**Tableau 1**

| | | | | | | |
|---|---|---|---|---|---|---|
| Durée Incubation HCℓ O.1N | T0 | T 5 min | T 10 min | T 20 min | T 30 min | T 60 min |
| Pancréatine | 100% | 60% | 5% | 0% | 0% | 0% |
| Formulation exemple 4 | 100% | 100% | 101% | 96% | 97% | 95% |

La pancréatine formulée selon l'invention conserve au moins 95% de son activité lipasique au bout d'une heure d'incubation à 37°C en milieu acide chlorhydrique 0.1N sous agitation (30 rpm).

Essai de vitesse de libération des lipases en condition de pH intestinal. On mesure l'activité lipasique par hydrolyse des acides gras libérés à partir de triglycérides de l'huile d'olive par titrage avec une solution (NaOH 0.02N) à pH constant de 9,0 et à 37°C. Le substrat est une solution d'huile d'olive pharmacopée, préparée selon le protocole "FIP lipase" adapté pour la préparation de 400 ml :
- Substrat 240 ml, dont 24,5% d'huile d'olive émulsionnée à l'aide de gomme arabique ;
- H₂Od : 140 ml
- Solution taurocholate de sodium (0.5%) : 20 ml
- quantité de pancréatine : 500 UI.

Les résultats sont rassemblés dans le tableau 2 ci-dessous. Les résultats sont exprimés en millimoles (mmol) d'acides gras libérés.

**Tableau 2**

| | | | | | | | | |
|---|---|---|---|---|---|---|---|---|
| Durée (Min) | 0 | 2 | 5 | 10 | 20 | 40 | 60 | 120 |
| Pancréatine | 0 | 0 | 1 mmol | 4 mmol | 11 mmol | 37 mmol | 51 mmol | 52 mmol |
| Forulation exemple 4 | 0 | 0 | 1 mmol | 3 mmol | 9 mmol | 31 mmol | 42 mmol | 44 mmol |
| Creon 6000 | 0 | 0 | 0 | 1 mmol | 2 mmol | 7 mmol | 18 mmol | 36 mmol |

À 20 minutes la quantité d'acide gras libérée est quatre fois supérieure avec la formulation 5 selon l'invention qu'avec la formulation commerciale de pancréatine basée sur l'enrobage dit "enterocoating"

## Revendications

1. - Formulation d'au moins une enzyme digestive, la formulation comprenant des particules lipidiques solides,
les particules lipidiques solides étant :
- de taille comprise entre 50 µm et 1200 µm,
- strictement hydrophobes,
- exemptes d'eau, de solvant organique, de composé tensioactif et de polymère,
et comprenant :
- au moins une enzyme digestive,
- une matrice cireuse strictement hydrophobe, non hygroscopique,
dans lesquelles, ladite au moins une enzyme digestive :
- présente une répartition homogène dans chaque particule lipidique solide de la formulation,
- est répartie sans gradient de distribution vers l'intérieur de la matrice cireuse, et
- représente entre 0.1% et 90% de la masse de la formulation,
la formulation présentant un point de fusion compris entre 20°C et 65°C.

2. - Formulation selon la revendication 1, **caractérisée en ce que** les particules lipidiques solides présentent une taille comprise entre 150 µm et 800 µm, notamment comprise entre 250 µm et 550 µm.

3. - Formulation selon l'une quelconque des revendications 1 et 2 **caractérisée en ce que** le point de fusion est compris entre 20°C et 55°C.

4. - Formulation selon l'une quelconque des revendications 1 à 3, **caractérisée en ce que** la matrice cireuse est constituée d'au moins un corps gras choisi dans le groupe formé des cires végétales, des cires d'abeilles, des cires d'abeilles modifiées, de la paraffine et des dérivés de paraffine, d'ozokérite, des polyoléfines, des acides gras, des esters d'acides gras, des triglycérides, des dérivés de triglycérides, d'huile de palme et du beurre de cacao.

5. - Formulation selon l'une quelconque des revendications 1 à 4, **caractérisée en ce qu'**elle contient des acides gras ou des esters d'acides gras en proportion massique comprise entre 0,5% et 75% de la formulation.

6. - Formulation selon l'une quelconque des revendications 1 à 5, **caractérisée en ce que** chaque particule lipidique solide comprend une seule enzyme digestive.

7. - Formulation selon l'une quelconque des revendications 1 à 6, **caractérisée en ce qu'**elle est formé d'un mélange de particules lipidiques solides, chaque particule lipidique solide comprenant une seule enzyme digestive, le mélange de particules lipidiques solides formant un mélange d'enzymes digestives.

8. - Préparation galénique comprenant une formulation selon l'une quelconque des revendications 1 à 7, **caractérisée en ce qu'**elle contient au moins un composé additionnel choisi dans le groupe formé des agents de lubrification des agents d'homogénéisation, des liants pharmaceutiquement acceptables, des stabilisants, des délitants, des désintégrants, des colorants, des conservateurs, des édulcorants, des diluants, des épaississants.

9. - Préparation galénique selon la revendication 8, **caractérisé en ce que** ledit composé additionnel est en proportion massique comprise entre environ 0,1 et 99% dans la composition galénique.

10. - Préparation galénique selon l'une des revendications 8 ou 9, **caractérisée en ce qu'**elle se présente sous une forme choisie dans le groupe formé d'une poudre, d'un comprimé, d'une tablette, d'une gélule, d'une capsule.

11. - Procédé de préparation d'une formulation selon l'une quelconque des revendications 1 à 7, **caractérisé en ce qu'**il comprend les étapes suivantes :
- a/ fusion de la matrice cireuse sous agitation, puis diminution de la température de la matrice cireuse à une température supérieure d'au moins 3°C à la température de fusion de la matrice cireuse,
- b/ addition et dispersion de ladite au moins une enzyme digestive dans la matrice cireuse fondue,
- c/ solidification du mélange formé par refroidissement à une température inférieure d'au moins 15°C à la température de de fusion de la matrice cireuse,
- e/ broyage mécanique à une température inférieure d'au moins 10°C, et préférentiellement 20°C, au point de fusion du mélange solidifié, ce par quoi on forme la formulation de particules lipidiques solides sous forme de poudre.

12. - Procédé selon la revendication 11, **caractérisé en ce qu'**il comprend une étape d/ de prébroyage et qu'au moins l'une des étapes c/ de solidification, d/ de prébroyage et e/ de broyage est réalisée par refroidissement avec de la carboglace ou l'azote liquide.

## Patentansprüche

1. Formulierung mindestens eines Verdauungsenzyms, wobei die Formulierung feststoffliche lipidartige Partikel umfasst,
wobei die feststofflichen lipidartigen Partikel:
- eine Größe im Bereich von 50 µm bis 1200 µm haben,
- streng hydrophob sind,
- frei von Wasser, von organischen Lösungsmitteln, von grenzflächenaktiven Verbindungen und von Polymeren sind,
und Folgendes umfassen:
- mindestens ein Verdauungsenzym,
- eine streng hydrophobe wachsartige Matrix, die nicht hygroskopisch ist,
wobei das mindestens eine Verdauungsenzym:
- eine gleichförmige Verteilung in jedem der feststofflichen lipidartigen Partikel der Formulierung aufweist,
- derart verteilt ist, dass kein Verteilungsgradient zum Inneren der wachsartigen Matrix hin besteht, und
- zwischen 0,1 % und 90 % der Masse der Formulierung ausmacht,
wobei die Formulierung einen Schmelzpunkt im Bereich von 20 °C bis 65 °C aufweist.

2. Formulierung nach Anspruch 1, **dadurch gekennzeichnet, dass** die feststofflichen lipidartigen Partikel eine Größe im Bereich von 150 µm bis 800 µm, insbesondere im Bereich von 250 µm bis 550 µm aufweisen.

3. Formulierung nach einem beliebigen der Ansprüche 1 und 2, **dadurch gekennzeichnet, dass** der Schmelzpunkt im Bereich von 20 °C bis 55 °C liegt.

4. Formulierung nach einem beliebigen der Ansprüche 1 bis 3, **dadurch gekennzeichnet, dass** die wachsartige Matrix aus mindestens einem Fettstoff besteht, welcher aus der Gruppe ausgewählt ist, die von pflanzlichen Wachsen, Bienenwachs, modifiziertem Bienenwachs, Paraffin und Paraffinderivaten, Ozokerit, Polyolefinen, Fettsäuren, Fettsäureestern, Triglyceriden, Triglyceridderivaten, Palmöl und Kakaobutter gebildet wird.

5. Formulierung nach einem beliebigen der Ansprüche 1 bis 4, **dadurch gekennzeichnet, dass** sie Fettsäuren oder Fettsäureester zu einem Massenanteil enthält, der im Bereich von 0,5 % bis 75 % der Formulierung liegt.

6. Formulierung nach einem beliebigen der Ansprüche 1 bis 5, **dadurch gekennzeichnet, dass** jeder der feststofflichen lipidartigen Partikel nur ein Verdauungsenzym umfasst.

7. Formulierung nach einem beliebigen der Ansprüche 1 bis 6, **dadurch gekennzeichnet, dass** sie aus einer Mischung feststofflicher lipidartiger Partikel gebildet ist, wobei jeder der feststofflichen lipidartigen Partikel nur ein Verdauungsenzym umfasst, wobei die Mischung feststofflicher lipidartiger Partikel eine Mischung von Verdauungsenzymen bildet.

8. Galenische Zubereitung, die eine Formulierung gemäß einem beliebigen der Ansprüche 1 bis 7 umfasst, **dadurch gekennzeichnet, dass** sie mindestens eine zusätzliche Verbindung enthält, welche aus der Gruppe ausgewählt ist, die von den Gleitmitteln, den Homogenisierungsmitteln, den pharmazeutisch unbedenklichen Bindemitteln, den Stabilisatoren, den zerfallsfördernden Mitteln, den Sprengmitteln, den Farbstoffen, den Konservierungsstoffen, den Süßungsmitteln, den Verdünnungsmitteln, den Verdickungsmitteln gebildet wird.

9. Galenische Zubereitung nach Anspruch 8, **dadurch gekennzeichnet, dass** die zusätzliche Verbindung zu einem Massenanteil in der galenischen Zusammensetzung vorliegt, der im Bereich von 0,1 bis 99 % liegt.

10. Galenische Zubereitung nach einem der Ansprüche 8 oder 9, **dadurch gekennzeichnet, dass** sie in einer Form vorliegt, welche aus der Gruppe ausgewählt ist, die von einem Pulver, einer Tablette, einem im Mund zerfallenden Plättchen, einer Gelkapsel, einer Kapsel gebildet wird.

11. Verfahren zur Herstellung einer Formulierung nach einem beliebigen der Ansprüche 1 bis 7, **dadurch gekennzeichnet, dass** es die folgenden Schritte umfasst:
- a/ Schmelzen der wachsartigen Matrix unter Rühren, woraufhin die Temperatur der wachsartigen Matrix auf eine Temperatur gesenkt wird, welche mindestens 3 °C höher als die Schmelztemperatur der wachsartigen Matrix ist,
- b/ Zusetzen des mindestens einen Verdauungsenzymes und Dispergieren desselben in der geschmolzenen wachsartigen Matrix,
- c/ Erstarrenlassen der entstandenen Mischung, indem diese auf eine Temperatur abgekühlt wird, die mindestens 15 °C niedriger als die Schmelztemperatur der wachsartigen Matrix ist,
e/ Mechanisches Zerkleinern bei einer Temperatur, die mindestens 10 °C, und vorzugsweise 20 °C, niedriger als der Schmelzpunkt der erstarrten Mischung ist, wobei auf diese Weise die Formulierung feststofflicher lipidartiger Partikel in Pulverform gebildet wird.

12. Verfahren nach Anspruch 11, **dadurch gekennzeichnet, dass** es einen Schritt d/ des Vorzerkleinern umfasst und dass mindestens einer der Schritte c/ des Erstarrenlassens, d/ des Vorzerkleinerns und e/ des Zerkleinerns durchgeführt wird, indem eine Kühlung mit Trockeneis oder flüssigem Stickstoff erfolgt.

## Claims

1. - Formulation of at least one digestive enzyme, the formulation comprising solid lipid particles,
the solid lipid particles being:
- between 50 µm and 1200 µm in size,
- strictly hydrophobic,
- free of water, of organic solvent, of surfactant compound and of polymer,
and comprising:
- at least one digestive enzyme,
- a strictly hydrophobic, non-hygroscopic waxy matrix,
in which said at least one digestive enzyme:
- has homogeneous distribution in each solid lipid particle of the formulation,
- is distributed without a distribution gradient towards the interior of the waxy matrix, and
- represents between 0.1% and 90% of the mass of the formulation,
the formulation having a melting point of between 20°C and 65°C.

2. - Formulation according to Claim 1, **characterized in that** the solid lipid particles have a size of between 150 µm and 800 µm and notably between 250 µm and 550 µm.

3. - Formulation according to either of Claims 1 and 2, **characterized in that** the melting point is between 20°C and 55°C.

4. - Formulation according to any one of Claims 1 to 3, **characterized in that** the waxy matrix consists of at least one fatty substance chosen from the group formed from plant waxes, beeswaxes, modified beeswaxes, paraffin and paraffin derivatives, ozokerite, polyolefins, fatty acids, fatty acid esters, triglycerides, triglyceride derivatives, palm oil and cocoa butter.

5. - Formulation according to any one of Claims 1 to 4, **characterized in that** it contains fatty acids or fatty acid esters in a mass proportion of between 0.5% and 75% of the formulation.

6. - Formulation according to any one of Claims 1 to 5, **characterized in that** each solid lipid particle comprises only one digestive enzyme.

7. - Formulation according to any one of Claims 1 to 6, **characterized in that** it is in the form of a mixture of solid lipid particles, each solid lipid particle comprising only one digestive enzyme, the mixture of solid lipid particles forming a mixture of digestive enzymes.

8. - Galenical preparation comprising a formulation according to any one of Claims 1 to 7, **characterized in that** it contains at least one additional compound chosen from the group formed from lubricants, homogenizing agents, pharmaceutically acceptable binders, stabilizers, breakdown agents, disintegrants, colorants, preserving agents, sweeteners, diluents and thickeners.

9. - Galenical preparation according to Claim 8, **characterized in that** said additional compound is in a mass proportion of between about 0.1% and 99% in the galenical composition.

10. - Galenical preparation according to either of Claims 8 and 9, **characterized in that** it is in a form chosen from the group formed from a powder, a tablet, a lozenge, a gel capsule and a wafer capsule.

11. - Process for preparing a formulation according to any one of Claims 1 to 7, **characterized in that** it comprises the following steps:
- a/ melting of the waxy matrix with stirring, followed by reducing the temperature of the waxy matrix to a temperature at least 3°C above the melting point of the waxy matrix,
- b/ addition and dispersion of said at least one digestive enzyme in the molten waxy matrix,
- c/ solidification of the mixture formed by cooling to a temperature at least 15°C below the melting point of the waxy matrix,
- e/ mechanical milling at a temperature at least 10°C and preferentially 20°C below the melting point of the solidified mixture, whereby the formulation of solid lipid particles in powder form is formed.

12. - Process according to Claim 11, **characterized in that** it comprises a premilling step d/ and **in that** at least one step from among the solidification step c/, the premilling step d/ and the milling step e/ is performed by cooling with cardice or with liquid nitrogen.
